(19) Europäisches Patentamt
European Patent Office
Office européen des brevets

(11) Veröffentlichungsnummer: **0 005 748**
**B1**

# (12) EUROPÄISCHE PATENTSCHRIFT

(45) Veröffentlichungstag der Patentschrift: **29.12.82**

(51) Int. Cl.³: **C 07 C 175/00** // C07F9/54, C07C49/713, C07D317/64

(21) Anmeldenummer: **79101400.4**

(22) Anmeldetag: **08.05.79**

(54) **Verfahren zur Herstellung von Cyclohexenderivaten sowie Zwischenprodukte in diesem Verfahren.**

(30) Priorität: **02.06.78 CH 6074/78**
**29.03.79 CH 2922/79**

(43) Veröffentlichungstag der Anmeldung:
**12.12.79 Patentblatt 79/25**

(45) Bekanntmachung des Hinweises auf die Patenterteilung:
**29.12.82 Patentblatt 82/52**

(84) Benannte Vertragsstaaten:
**AT BE CH DE FR GB IT NL**

(56) Entgegenhaltungen:
**FR-A-2 357 541**
**HELVETICA CHIMICA ACTA, Vol. 61, Nr. 7, November 1978, Basel, CH. F. KIENZLE et al.: »Synthese von optisch aktiven, natürlichen Carotinoiden und strukturell verwandten Naturprodukten. II. Synthese von (3,S,3'S9)-Abstaxanthin«, Seiten 2609–2615.**

(73) Patentinhaber: **F. HOFFMANN-LA ROCHE & CO. Aktiengesellschaft, CH-4002 Basel (CH)**

(72) Erfinder: **Broger, Emil Albin, Dr., Obere Egg 11, CH-4465 Magden (CH)**
Erfinder: **Crameri, Yvo, Dr., Binningerstrasse 28, CH-4104 Oberwil (CH)**
Erfinder: **Leuenberger, Hans Georg Wilhelm, Dr., Rebenstrasse 23, CH-4112 Bättwil (CH)**
Erfinder: **Widmer, Erich, Dr., Mittelweg 47, CH-4142 Münchenstein (CH)**
Erfinder: **Zell, Reinhard, Dr., Im Zwären, CH-4118 Rodersdorf (CH)**

(74) Vertreter: **Aschert, Hubert et al, Postfach 601 Grenzacherstrasse 124, CH-4002 Basel (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 005 748 B1

BUNDESDRUCKEREI BERLIN

Verfahren zur Herstellung von Cyclohexenderivaten, sowie Zwischenprodukte in diesem Verfahren

Die vorliegende Erfindung betrifft ein Verfahren zur Herstellung von Cyclohexenderivaten, nämlich von racemischen und optisch aktiven Polyenverbindungen, und zwar von racemischem und optisch aktivem Astaxanthin und dafür geeigneten Zwischenprodukten, sowie auch bestimmte neue Zwischenprodukte in diesem Verfahren.

Das erfindungsgemäße Verfahren ist dadurch gekennzeichnet, daß man ein racemisches oder optisch aktives Diol der Formel

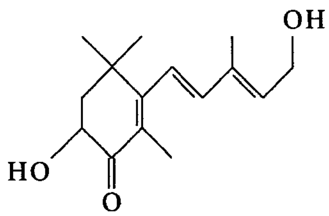

I

halogeniert und das erhaltene Halogenid mit einem Triarylphosphin umsetzt, und daß man erwünschtenfalls das erhaltene Phosphoniumsalz der allgemeinen Formel

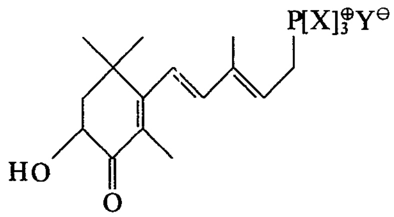

III

in der X einen Arylrest und Y Halogen bezeichnet, mit dem Dialdehyd der Formel

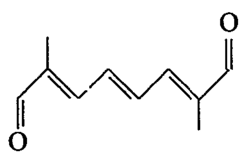

IV

zu racemischem oder optisch aktivem Astaxanthin kondensiert.

Bei den unter den Ausdrücken »halogenieren«, »Halogenid« und »Halogen« angesprochenen Halogenen handelt es sich um Chlor, Brom und Jod.

In den obigen, sowie in folgenden Formeln ist jeweils die all-trans-Konfiguration dargestellt. Die Erfindung umfaßt jedoch auch mögliche cis-Konfigurationen.

Die Halogenierung der Verbindungen der Formel I kann in an sich bekannter Weise mittels Halogenwasserstoff (Chlorwasserstoff, Bromwasserstoff bzw. Jodwasserstoff), und zwar in wäßriger Lösung (z. B. 37%, 48% oder 57%) erfolgen. Die Halogenierung kann bei Temperaturen zwischen etwa −10° C und +10° C erfolgen, vorzugsweise bei etwa 0° C. Als Lösungsmittel für die Durchführung der Reaktion kann ein für derartige Halogenierungen geeignetes Lösungsmittel verwendet werden, beispielsweise ein chlorierter Kohlenwasserstoff, wie Methylenchlorid oder Chloroform.

Die Umsetzung des so erhaltenen Halogenids mit einem Triarylphosphin, insbesondere mit Triphenylphosphin, zum Phosphoniumsalz der Formel III kann in an sich bekannter Weise, z. B. in Äthylacetat, vorzugsweise in einer Inertatmosphäre, und in Gegenwart eines säurebindenden Mittels, z. B. eines Alkylenoxids, wie 1,2-Butylenoxid, durchgeführt werden, und zwar bei etwa Raumtemperatur bzw. bei erhöhter Temperatur.

Die Umsetzung des erhaltenen Phosphoniumsalzes der Formel III mit dem Dialdehyd der Formel IV kann ebenfalls in einem geeigneten Lösungsmittel, wie z. B. Methylenchlorid oder Chloroform, und in Gegenwart eines säurebindenden Mittels, z. B. Butylenoxid oder Natriummethylat, erfolgen.

Das auf diese Weise erhaltene Astaxanthin liegt, je nachdem, ob racemisches oder optisch aktives Ausgangsmaterial verwendet wird, in Form des Racemats [(3RS,3′RS)-Konfiguration] oder in optisch aktiver Form [(3S,3′S)-Konfiguration oder (3R,3′R)-Konfiguration] oder in meso-Form vor.

In der FR-A-2 357 541 wird die Halogenierung von Cyclohexenverbindungen beschrieben, welche sich von der Cyclohexenverbindung der obigen Formel I dadurch unterscheiden, daß sie anstelle der Hydroxygruppe am Cyclohexenring eine Acyloxygruppe tragen. Bei dem dort beschriebenen Halogenierungsverfahren dient die Acylgruppe als Schutzgruppe, um zu verhindern, daß bei der Halogenierung (Ersatz der Hydroxygruppe in der Seitenkette durch Halogen) auch ein Ersatz der Hydroxygruppe am Cyclohexenring durch Halogen eintritt.

Unerwarteterweise wurde nun im Rahmen der vorliegenden Erfindung festgestellt, daß die Gegenwart einer Schutzgruppe nicht erforderlich ist und daß das Diol der Formel I halogeniert werden kann, wobei lediglich die Hydroxygruppe in der Seitenkette, nicht jedoch die Hydroxygruppe am Cyclohexenring durch Halogen ersetzt wird. Dies bedeutet im Hinblick auf die Vermeidung der Einführung einer Schutzgruppe eine bedeutende Verfahrensvereinfachung.

Die vorliegende Erfindung betrifft auch bestimmte für das oben beschriebene Astaxanthinverfahren geeignete Zwischenprodukte, nämlich Cyclohexanonverbindungen der Formel

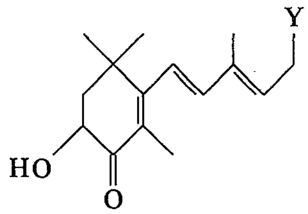

II

worin Y Halogen bedeutet, in racemischer Form und in Form der optischen Antipoden.

Diese Cyclohexanonverbindungen können dadurch erhalten werden, daß man 2-Hydroxyketoisophoron der Formel

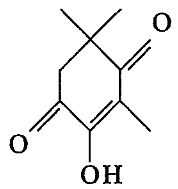

V

zu einem Diol der Formel

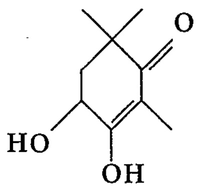

VI

reduziert, daß man im erhaltenen Diol der Formel VI die freien Hydroxygruppen in geschützte Hydroxygruppen überführt, daß man das erhaltene Produkt gegebenenfalls alkinyliert und zum racemischen oder optisch aktiven Acetylendiol der Formel

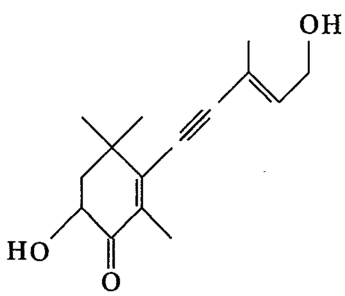

VII

umlagert, dieses partiell zum Diol der Formel

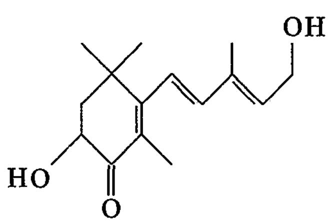

I

reduziert und das erhaltene Diol der Formel I, wie oben angegeben, halogeniert.

In der ersten Stufe dieses Verfahrens wird das 2-Hydroxyketoisophoron der Formel V selektiv reduziert. Diese Reduktion erfolgt zweckmäßig in der Form, daß man die Verbindung der Formel V in einem geeigneten Lösungsmittel, und zwar in wäßrig-alkalischer Lösung, in Gegenwart von

Raney-Nickel mit Wasserstoff behandelt. Es bildet sich dabei zuerst das Alkalimetallsalz und dieses wird reduziert. Diese Reduktion kann beispielsweise bei Raumtemperatur durchgeführt werden.

Als Reduktionsprodukt erhält man ein Tautomerengemisch, bestehend aus den Verbindungen der Formeln

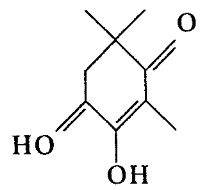

VI

und

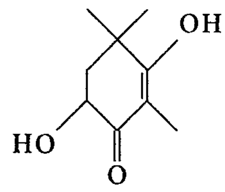

VIa

von welchen die Verbindung der Formel VI die für das weitere Verfahren geeignete Verbindung darstellt.

Zwecks Blockierung der gewünschten tautomeren Form in der Verbindung der Formel VI und zum Schutz der beiden darin enthaltenen Hydroxygruppen werden letztere in geschützte Hydroxygruppen übergeführt. Dies erfolgt zweckmäßig durch Acetonisierung, vorzugsweise mit Aceton, wobei eine Verbindung der Formel

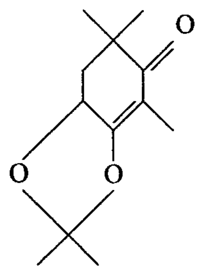

VIII

erhalten wird, in welcher die Hydroxygruppen der Verbindung der Formel VI in geschützter Form vorliegen.

Die Acetonisierung kann aber auch mittels 2,2-Dimethoxypropan oder einem Isopropenylalkyläther, wie Isopropenylmethyläther, erfolgen.

Die Acetonisierung kann zweckmäßig in an sich bekannter Weise unter den Bedingungen einer Säurekatalyse und unter Wasserabscheidung erfolgen. Dabei wird die im Tautomerengemisch aus der vorhergehenden Reduktion vorhandene Verbindung VIa im Verlaufe der Acetonisierungsreaktion völlig in das Tautomere VI übergeführt und schließlich das ganze Material als Acetonid VIII fixiert.

Der Schutz der Hydroxygruppen im Reduktionsprodukt VI kann aber auch durch Dimerisierung des Diols VI erfolgen, und zwar zweckmäßig ebenfalls durch Säurekatalyse unter Wasserabscheidung, beispielsweise durch Kochen mit p-Toluolsulfonsäure oder Perchlorsäure in einem geeigneten Lösungsmittel, beispielsweise in Toluol. Es werden hierbei die Hydroxygruppen der Verbindung VI unter intermolekularer Ätherbildung und Bildung eines Dimeren geschützt. Auch hierbei erfolgt eine vollständige Überführung des tautomeren Diols VIa in das Dimere und damit Fixierung der gewünschten tautomeren Form.

Die in der nächsten Stufe erfolgende Alkinylierung erfolgt zweckmäßig unter Bedingungen, bei welchen eine Dehydratisierung des Alkinylierungsproduktes und Abspaltung der Schutzgruppe erfolgt, und zwar unter den Bedingungen einer sauren Hydrolyse des Alkinylierungsproduktes.

Die Alkinylierung kann mit einem gegebenenfalls geschützten 3-Methylpenteninol, beispielsweise mit einem Silyläther von 3-Methylpentenin-1- oder 3-ol der Formeln

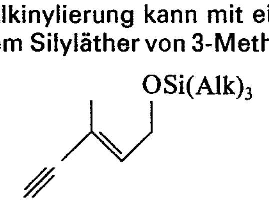

oder

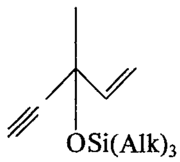

worin Alk eine niedere Alkylgruppe, beispielsweise Methyl, bedeutet, oder mit Aceton-3-methyl-2-penten-4-inyl-acetal oder mit tert. Butyl-3-methyl-2-penten-4-inyläther, und zwar unter für solche Alkinylierungen an sich bekannten Bedingungen erfolgen.

Das durch die Alkinylierung, Dehydratisierung und Schutzgruppenabspaltung erhaltene Acetylendiol der Formel VII wird in der nächsten Stufe zum Diol I reduziert.

Diese Partialhydrierung erfolgt zweckmäßig mit Zink und Eisessig.

Als Lösungsmittel für die Hydrierungsreaktion kann ein geeignetes organisches Lösungsmittel, beispielsweise ein chlorierter Kohlenwasserstoff, wie Methylenchlorid, oder aber der als Reagens verwendete Eisessig verwendet werden.

Vorzugsweise verwendet man eine etwa 2%ige Lösung des Ausgangsmaterials in Methylenchlorid/Eisessig, wobei man letzteren im Verhältnis von etwa 1 : 2 bis 1 : 2 · 5 anwendet. Das Zink wird zweckmäßig in einer Menge von etwa 1—3 Grammatom, vorzugsweise 2,5 Grammatom, pro Mol Ausgangsmaterial verwendet.

Die Hydrierungsreaktion kann bei Temperaturen zwischen etwa —20° C und etwa Raumtemperatur durchgeführt werden. Vorzugsweise wird die Hydrierung bei etwa 0° C durchgeführt.

Die erhaltene Verbindung der Formel I kann dann, wie weiter oben beschrieben, über die Verbindung III in Astaxanthin übergeführt werden.

Zwecks Herstellung der optisch aktiven Polyenverbindungen wird erfindungsgemäß das racemische Diol der Formel VI mit einer Spaltbase in die optisch aktiven Formen aufgespalten und es werden diese optisch aktiven Formen den weiteren Verfahrensschritten (VI → VIII → VII → I → III → Astaxanthin) unterworfen.

Als Spaltbase wird hierbei zweckmäßig $\alpha$-Phenyläthylamin verwendet, und zwar entweder in Form des (S)-(—)-$\alpha$-Phenyläthylamins oder des (R)-(+)-$\alpha$-Phenyläthylamins.

Das für die Herstellung von natürlichem, im Lachs vorkommendem Astaxanthin erforderliche optisch aktive Ausgangsmaterial der Formel

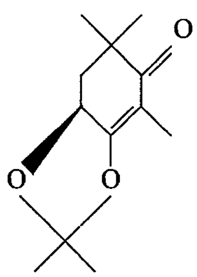

VIIIa

kann aber auch dadurch erhalten werden, daß man die Verbindung der Formel V mit Hefen, beispielsweise mit Preßhefe, fermentativ reduziert, wobei man ein Gemisch der beiden tautomeren Diole VI und VIa in der der Verbindung der Formel VIIIa entsprechenden optisch aktiven Form erhält, welches Gemisch dann, wie oben für die Überführung des Gemisches von VI und VIa beschrieben, acetonisiert wird, wobei man die optisch aktive Verbindung der Formel VIIIa erhält, die dann über die entsprechenden optisch aktiven Formen von VII, I und III in das optisch aktive natürliche, im Lachs vorkommende Astaxanthin übergeführt wird.

## Beispiel 1

In einen 200-ml-Sulfierkolben, versehen mit Rührer, 50-ml-Tropftrichter mit Druckausgleich, Thermometer und einer Vorrichtung zur Inertbegasung wird eine Lösung von 25 g 6-Hydroxy-3-(5-hydroxy-3-methyl-1,3-pentadienyl)-2,4,4-trimethyl-2-cyclohexen-1-on in 150 ml Methylenchlorid eingebracht. Die erhaltene Lösung wird unter Argonatmosphäre mit einem Eisbad auf 0° bis 5° C gekühlt und anschließend das Reaktionsgemisch bis zur Aufarbeitung in diesem Temperaturbereich gehalten. So schnell es die Einhaltung dieser Reaktionstemperatur erlaubt, werden 30 ml Bromwasserstofflösung (63% in Wasser) zugetropft. Das Reaktionsgemisch wird noch 5 Minuten nachgerührt und dann in einem 1-Liter-Scheidetrichter auf 100 ml 10prozentige Kochsalzlösung gegossen. Zusätzlich werden noch 400 ml Äthylacetat in den Scheidetrichter gegeben und dann das Ganze gut geschüttelt. Die nun untenstehende wäßrige Phase wird abgetrennt und verworfen. Die organische Phase wird in 2

Portionen mit insgesamt 200 ml 5prozentiger Natriumbicarbonatlösung gewaschen. Die Waschlösungen werden verworfen. Die organische Phase wird mit 1 ml 1,2-Butylenoxid versetzt, mit 50 g Natriumsulfat (wasserfrei) getrocknet, filtriert, das Trocknungsmittel auf dem Filter mit 100 ml Äthylacetat nachgewaschen und die Bromidlösung anschließend im Rotationsverdampfer am Wasserstrahlvakuum bei einer Badtemperatur von 30° C auf etwa 250 ml eingeengt. Das Partialvakuum im Rotationsverdampfer wird mit Stickstoff aufgehoben und das erhaltene gelbe Konzentrat umgehend für die Darstellung des Phosphoniumsalzes eingesetzt.

In einem 1,5-Liter-Sulfierkolben, versehen mit Rührer, Thermometer, 250-ml-Tropftrichter mit Druckausgleich und einer Vorrichtung zur Inertbegasung, wird eine Lösung von 30 g Triphenylphosphin und 1 ml 1,2-Butylenoxid in 250 ml Äthylacetat eingebracht. Das Reaktionsgefäß wird während der ganzen Reaktionsdauer mit Argon begast. Bei Raumtemperatur wird nun im Verlaufe von etwa 2 Stunden die nach den obigen Angaben hergestellte Bromidlösung zugetropft.

Beim Auftreten der ersten Trübungserscheinungen im Reaktionsgemisch werden Impfkristalle zugesetzt. Das weiße Phosphoniumsalz kristallisiert darauf laufend aus, und die Temperatur des Reaktionsgemisches steigt leicht — bis auf etwa 28° C — an. Nach beendeter Zugabe des Bromids wird noch weitere 24 Stunden gerührt und dann das Produkt unter Stickstoff abfiltriert. Das weiße Kristallisat wird mit 250 ml Äthylacetat gewaschen und dann bei 40° C im Trockenschrank am Wasserstrahlvakuum bis zur Gewichtskonstanz getrocknet.

Man erhält 54,1 g [(4E)-5-(4-Hydroxy-2,6,6-trimethyl-3-oxo-1-cyclohexen-1-yl)-3-methyl-2,4-pentadienyl]-triphenyl-phosphoniumbromid mit einem Schmelzpunkt von 172 – 174° C.

In einem 500-ml-Sulfierkolben, versehen mit Rührer, 50-ml-Tropftrichter mit Druckausgleich, Thermometer und einer Vorrichtung zur Inertbegasung werden unter Rühren und Begasung mit Argon in 250 ml Methylenchlorid 69 g des nach den obigen Angaben erhaltenen Phosphoniumbromids und 8,2 g 2,7-Dimethyl-octatrien-(2,4,6)-dial-(1,8) gelöst. Diese Lösung wird unter fortgesetztem Rühren mit einem Eisband auf 0 – 5° C gekühlt und bei dieser Temperatur im Verlaufe von 90 Minuten tropfenweise mit 27,6 ml Natriummethylatlösung (enthaltend 6,48 g Natriummethylat) versetzt. Eine Stunde danach wird das Kühlbad entfernt und noch 18 Stunden bei Raumtemperatur reagieren gelassen.

Nach Aufarbeitung mit Methylenchlorid, Isomerisierung durch Erhitzen in Heptan und weiterer Reinigung mit Methylenchlorid und Methanol erhält man 23,1 g rac. Astaxanthin mit einem Schmelzpunkt von 220 – 222° C.

Das bei diesem Verfahren als Ausgangsmaterial verwendete 6-Hydroxy-3-(5-hydroxy-3-methyl-1,3-pentadienyl)-2,4,4-trimethyl-2-cyclohexen-1-on kann wie folgt erhalten werden:

In einer 3 Liter Hydrierapparatur mit Rührer werden 40 g Natriumhydroxid in 1,1 Liter Wasser gelöst und die auf 20° C gekühlte Lösung wird mit 168,2 g 2-Hydroxy-3,5,5-trimethyl-2-cyclohexen-1,4-dion (2-Hydroxy-ketoisophoron), und 28 g Raney-Nickel versetzt.

In der Apparatur wird nun die Luft durch Wasserstoff ersetzt und die Reaktion unter starkem Rühren gestartet. Nach 4 Stunden bzw. nach Aufnahme von etwa 25 Litern Wasserstoff kommt die Wasserstoffaufnahme zum Stillstand und nach einer weiteren Stunde wird der Katalysator vom Reaktionsgemisch abfiltriert und mit Wasser nachgewaschen. Das Gesamtfiltrat wird mit 90 ml 37prozentiger Salzsäure angesäuert und in Scheidetrichtern zwischen gesättigter Natriumchloridlösung und Methylenchlorid verteilt. Die vereinigten Methylenchloridextrakte werden über Natriumsulfat getrocknet und im Rotationsverdampfer am Wasserstrahlvakuum bei einer Badtemperatur von etwa 50° C bis zur Gewichtskonstanz eingedampft. Der Rückstand, ein Gemisch der beiden tautomeren Formen VI und VIa (3,4-Dihydroxy-2,6,6-trimethyl-2-cyclohexen-1-on und 3,6-Dihydroxy-2,4,4-trimethyl-2-cyclohexen-1-on) kann direkt in der nachfolgenden Acetonisierungsstufe eingesetzt werden:

Hierzu werden 200 g dieses Tautomerengemisches in einer mit einem Rührer, einem Wasserabscheider und einem mit Molekularsieb IG-2 (4 A) beschickten Trockenturm in 1,5 Liter Aceton eingebracht und unter Rühren bei Raumtemperatur tropfenweise mit 2 ml 70prozentiger Perchlorsäure versetzt. Nach einer Reaktionszeit von 6 – 7 Stunden bei 30° C werden der orangefarbenen Reaktionslösung 25 g pulverisiertes, wasserfreies Kaliumcarbonat zugesetzt.

Anschließend wird die Suspension bei Raumtemperatur und unter Feuchtigkeitsausschluß während 1 Stunde gerührt und dann mit 2 ml Pyridin versetzt. Die Festkörper werden über einer Glasfilternutsche abfiltriert und auf der Nutsche mit 2 Portionen Aceton zu je 50 ml, d. h. total mit 100 ml Aceton gewaschen. Die vereinigten Filtrate werden im Rotationsverdampfer am Wasserstrahlvakuum bei einer Badtemperatur von 30° C eingedampft. Der Rückstand (ein gelbes Öl) wird mit 600 ml n-Hexan und 100 ml 1N Natriumcarbonatlösung versetzt, durch kräftiges Schütteln in den beiden Phasen gelöst und das Ganze anschließend in Scheidetrichtern zwischen Hexan und einer wäßrigen Phase, bestehend aus 1N Natriumcarbonatlösung und gesättigter Natriumchloridlösung, verteilt. Die organischen Phasen werden vereinigt, über Natriumsulfat getrocknet und im Rotationsverdampfer am Wasserstrahlvakuum bei einer Badtemperatur von 40° C auf eine etwa 50prozentige Lösung eingeengt. Die blaßgelbe Lösung wird in einem 750-ml-Sulfierkolben (versehen mit Rührer, Thermometer und Chlorcalciumrohr) unter Rühren mit einem Aceton/Trockeneis-Bad auf −30° C gekühlt und bei dieser Temperatur während 5 Stunden belassen. Schon beim Abkühlen

beginnt das Produkt in schweren, weißen Kristallen auszufallen.

Die Kristalle wurden abgenutscht, auf der Nutsche mit 150 ml n-Hexan (auf −35°C gekühlt) gewaschen und anschließend bei Raumtemperatur im Trockenschrank am Vakuum (0,2 Torr) bis zur Gewichtskonstanz getrocknet. Man erhält 202 g Produkt mit einem Schmelzpunkt von 58−59°C. Aus der Mutterlauge werden durch Destillation und Kristallisation des Destillats weitere 29,6 g erhalten, so daß die Gesamtausbeute an rac. 7,7a-Dihydro-2,2,4,6,6-pentamethyl-1,3-benzo-dioxol-5(6H)-on 231,6 g beträgt.

In einen 10-Liter-Sulfierkolben, versehen mit Rührer, Thermometer, Kühler mit gefülltem Chlorcalciumrohr und 1-Liter-Tropftrichter mit Druckausgleich und Argonbegasung, werden 60,8 g Magnesium-Drehspäne und 550 ml abs. Tetrahydrofuran eingebracht. Dazu wird innert 50 Minuten unter Rühren eine Lösung von 226 ml Äthylbromid in 420 ml abs. Tetrahydrofuran getropft. Nach etwa 1 Minute startet die stark exotherme Grignard-Reaktion bei Raumtemperatur spontan. Die Zutropfgeschwindigkeit wird so eingestellt, daß mit Hilfe eines Eisbades die Temperatur zwischen 50−60°C gehalten werden kann.

Im Anschluß daran wird das Reaktionsgemisch mit einem Ölbad (Badtemperatur = 80°C) erwärmt und 90 Minuten nachgerührt.

Danach wird unter fortgesetztem Rühren die Temperatur des Reaktionsgemisches auf 40°C eingestellt und dann im Verlaufe von 65 Minuten eine Lösung von 500 ml Trimethyl-[(trans-3-methyl-pent-2-en-4-in)-oxy]-silan (1″-Pentol-silyläther), in 750 ml abs. Tetrahydrofuran zugetropft.

Unter Beibehaltung der beschriebenen Reaktionsbedingungen wird das Reaktionsgemisch während 2¹/₂ Stunden belassen und anschließend im Verlaufe von 30 Minuten eine Lösung von 210,3 g des nach den obigen Angaben erhaltenen Acetonids [rac. 7,7a-Dihydro-2,2,4,6,6-pentamethyl-1,3-benzodioxol-5(6H)-on] in 420 ml abs. Tetrahydrofuran zugetropft.

Während weiteren 16 Stunden werden die Reaktionsbedingungen (Rühren, 40°C, Argonbegasung) beibehalten und dann der auf Raumtemperatur abgekühlten Lösung 500 ml Äther zugesetzt.

Das Reaktionsgemisch wird einem Gemisch von 1000 g zerkleinertem Eis und 111 ml 96prozentige Schwefelsäure zugesetzt und 1 Minute lang intensiv gerührt.

Die wäßrige Phase wird zwischen Äther und gesättigter Natriumbicarbonat- und gesättigter Kochsalzlösung verteilt. Die organischen Phasen werden vereinigt, über Natriumsulfat getrocknet und im Rotationsverdampfer am Wasserstrahlvakuum bei einer Badtemperatur von etwa 50°C bis zur Gewichtskonstanz eingedampft. Man erhält 420 g eines Rohproduktes in Form eines braunen Öls, das nach Reinigung mit Isopropyläther und Hexan 189 g 6-Hydroxy-3-(5-hydroxy-3-methyl-3-penten-1-inyl)-2,4,4-trimethyl-2-cyclohexen-1-on in Form von schwach-gelben Kristallen mit einem Schmelzpunkt von 84−86°C ergibt.

In der nächsten Stufe wird das so erhaltene Ketodiol (150 g) in einem 10-Liter-Sulfierkolben (mit Rührer, Thermometer, Chlorcalciumrohr und einer Vorrichtung zur Inertbegasung) unter Rühren und Argonbegasung in 4,5 Liter Methylenchlorid und 0,975 Liter Eisessig gelöst. Die Lösung wird auf 0°C gekühlt und mit 75 g Zinkstaub versetzt.

Bei fortgesetztem Rühren unter Argon bei 0°C werden nach 3 Stunden nochmals 25 g Zinkstaub zugefügt und nach weiteren 2 Stunden aufgearbeitet.

Das noch kalte Reaktionsgemisch wird über einer Glassinternutsche filtriert und der Rückstand auf der Nutsche in 2 Portionen mit insgesamt 600 ml Methylenchlorid gewaschen. Nach weiterer Aufarbeitung mit Methylenchlorid, Trocknen über Natriumsulfat, Filtrieren und Eindampfen bei 50°C bis zur Gewichtskonstanz erhält man 158,7 g eines braunen Öls, das nach Reinigung mit Äther und Hexan 120,6 g 6-Hydroxy-3-(5-hydroxy-3-methyl-1,3-pentadienyl)-2,4,4-trimethyl-2-cyclohexen-1-on in Form eines cis/trans-Isomerengemisches ergibt.

Nach Kristallisation aus Äther und Eindampfen der Mutterlauge erhält man 83 g des cis-Isomeren in Form von gelben Kristallen mit einem Schmelzpunkt von 92−94°C und 35,9 g eines Gemisches von cis- und trans-Isomeren (1 : 1) in Form eines gelben Öls.

Das nach der Hydrierung mit Zink und Eisessig erhaltene Isomerengemisch kann direkt der eingangs beschriebenen Halogenierung und Überführung in das Phosphoniumsalz unterworfen werden.

## Beispiel 2

In Analogie zu den entsprechenden Angaben in Beispiel 1 wird durch Umsetzung von 10 g 6-Hydroxy-3-(5-hydroxy-3-methyl-1,3-pentadienyl)-2,4,4-trimethyl-2-cyclohexen-1-on mit 16 ml 37%iger wäßriger Salzsäure in 60 ml Methylenchlorid in einem 250 ml Sulfierkolben das entsprechende Chlorid hergestellt, dieses mit Triphenylphospin zum [(4E)-5-(4-Hydroxy-2,6,6-trimethyl-3-oxo-1-cyclohexen-1-yl)-3-methyl-2,4-pentadienyl]-triphenylphosphoniumchlorid (Smp. 184−186°C) umgesetzt und letzteres, ebenfalls in Analogie zu Beispiel 1, mit 2,7-Dimethyloctatrien-(2,4,6)-dial-(1,8) zu rac. Astaxanthin umgesetzt.

## Beispiel 3

In zu Beispiel 1 und 2 analoger Weise enthält man rac. Astaxanthin über das [(4E)-5-(4-Hydroxy-2,6,6-trimethyl-3-oxo-1-cyclohexen-1-yl)-3-methyl-2,4-pentadienyl]-triphenylphosphoniumjodid (Smp. 196–198° C).

## Beispiel 4

Statt des im Beispiel 1 für die Alkinylierung des 7,7a-Dihydro-2,2,4,6,6-pentamethyl-1,3-benzodioxol-5(6H)-on verwendeten Silyläthers kann auch Aceton-methyl-3-methyl-2-penten-4-inyl-acetal (hergestellt aus 1''-Pentol und Isopropenylmethyläther) verwendet werden.

## Beispiel 5

Der Schutz der Hydroxygruppen im 3,4-Dihydroxy-2,6,6-trimethyl-2-cyclohexen-1-on kann auch durch Dimerisierung gemäß den folgenden Angaben erfolgen:

In einen 2-l-Sulfierkolben, ausgerüstet mit Rührer, Thermometer, Wasserabscheider und einer Vorrichtung zur Inertbegasung werden 120 g des Tautomerengemisches von VI und VIa in 1,4 Liter Toluol gelöst und mit 7 ml 70prozentiger Perchlorsäure versetzt.

Unter Rühren und Begasung mit Stickstoff wird so lange am Rückfluß (Wasserabscheider) gekocht (etwa 5 Stunden), bis mittels GC-Analyse kein Ausgangsmaterial mehr nachgewiesen werden kann. Noch stets unter Rühren und Inertbegasung wird das Reaktionsgemisch auf Raumtemperatur abgekühlt und dann mit 12 ml 28prozentiger Natronlauge schwach alkalisch gestellt. Nun wird das Gemisch in einen 3-l-Scheidetrichter gespült, in 2 Portionen mit total 600 ml Wasser gewaschen und schließlich im Rotationsverdampfer am Wasserstrahlvakuum bei einer Badtemperatur von 60° C bis zur Gewichtskonstanz eingedampft. Der gelbbraune, zähe Rückstand wird unter Erwärmen in 300 ml Aceton gelöst und die Lösung anschließend unter Rühren mit einem Eisbad auf 0° C gekühlt. Dabei beginnt das Produkt schnell zu kristallisieren. Es wird noch über Nacht im Eisschrank stehen gelassen und dann das Kristallisat abgenutscht. Die Kristalle werden auf der Nutsche in 2 Portionen mit total 50 ml Aceton (auf 0° C gekühlt) gewaschen und anschließend im Trockenschrank am Wasserstrahlvakuum bei 50° C bis zur Gewichtskonstanz getrocknet. Nach Aufarbeitung der Mutterlaugen erhält man 83,8 g des dimeren Äthers des Ausgangsmaterials in Form von Kristallen mit einem Schmelzpunkt von 206–209° C.

Dieser kann analog den Angaben in Beispiel 1 mit Silyläther alkinyliert und weiterverarbeitet werden.

## Beispiel 6

Zwecks Herstellung der optisch aktiven Polyenderivate, insbesondere der beiden optischen Antipoden von Astaxanthin, wird das rac. 3,4-Dihydroxy-2,6,6-trimethyl-2-cyclohexen-1-on nach den beiden folgenden Methoden a) und b) gespalten:

a) 40 g 3,4-Dihydroxy-2,6,6-trimethyl-2-cyclohexen-1-on (in Form des gemäß den Angaben in Beispiel 1 erhaltenen Tautomerengemisches) werden in 250 ml abs. Äther und 50 ml abs. Methanol gelöst. Unter Rühren wird eine Lösung von 28,7 g (R)-(+)-1-Phenyläthylamin in 25 ml abs. Äther innerhalb von 20 Minuten zugetropft. Hierbei fällt allmählich ein Öl aus und die Reaktionstemperatur steigt auf 26° C.
Es wird 16 Stunden weitergerührt. Während dieser verschwindet das ausgefallene Öl und es bildet sich eine Suspension eines gelb gefärbten, lockeren Pulvers. Dieses wird mit vier Portionen zu je 150 ml abs. Äther gut gewaschen, wobei der zunächst klebrige Filterrückstand allmählich fest wird. Der Filterrückstand wird bei 70° C im Rotationsverdampfer in 400 ml Äthylacetat gelöst, angeimpft und dann bei ausgeschaltetem Heizbad über Nacht im rotierenden Verdampfer belassen. Das farblose Kristallisat wird abfiltriert und mit drei Portionen zu je 100 ml Äthylacetat gewaschen. Der Filterrückstand wird im Vakuumtrockenschrank bei 50° C über Nacht getrocknet. In einem 500-ml-Scheidetrichter werden 14,57 g des so erhaltenen diastereomeren Salzes, gelöst in 100 ml entonisiertem Wasser und 100 ml Methylenchlorid, eingebracht. Nach Zusatz von 20 ml 3N Schwefelsäure wird 1 Minute kräftig durchgeschüttelt. Die abgetrennte wäßrige Phase wird noch 2mal mit je 100 ml Methylenchlorid nachextrahiert. Die vereinigten Methylenchlorid-Phasen werden mit 25 ml gesättigter Kochsalzlösung gewaschen, über 20 g Natriumsulfat getrocknet, filtriert und das Trocknungsmittel auf der Nutsche 2mal mit je 50 ml Methylenchlorid nachgewaschen. Das Filtrat wird im Rotationsverdampfer bei einer Badtemperatur von 40° C bis zur Gewichtskonstanz eingedampft.

Das so erhaltene (3S)-Ketodiol ($[\alpha]_D = -27{,}2°$, 0,59% in Methanol) kann in Analogie zu den entsprechenden Angaben für das rac. Ketodiol in Beispiel 1 zum (3S,3'S)-Astaxanthin (in der Natur im Lachs vorkommend) weiterverarbeitet werden.

b) 40 g 3,4-Dihydroxy-2,6,6-trimethyl-2-cyclohexen-1-on (in Form des gemäß den Angaben in Beispiel 1 erhaltenen Tautomerengemisches) werden in 250 ml abs. Äther und 50 ml abs. Methanol gelöst. Die weitere Verarbeitung erfolgt in Analogie zu den Angaben in Teil a) und man erhält das (3R)-Ketodiol, $[\alpha]_D = +29{,}5°$ (1% in Methanol), das in Analogie zu den entsprechenden Angaben in Beispiel 1 in das (3R,3'R)-Astaxanthin (in der Natur in Hefen vorkommend) übergeführt werden kann.

Beispiel 7

In einem Fermenter wird eine aus 4,5 l entionisiertem Wasser, 500 g Preßhefe, 250 g Zucker, 10 g 2-Hydroxy-3,5,5-trimethyl-2-cyclohexen-1,4-dion und 1 g Antischaummittel (Polypropylenglykol-monobutyläther) bestehende Gärbrühe bei 30° C mit Oberflächenbelüftung (240 Liter/h) und unter Rühren (900 Umdrehungen/min) bebrütet. Nach 4 und nach 5 Tagen Fermentationszeit werden je 100 g Zucker zugegeben.

Nach 7 Tagen Fermentationszeit wird die Gärbrühe mit Kochsalz gesättigt und kontinuierlich mit Diäthyläther extrahiert. Nach Abdampfen des Äthers erhält man einen Rohextrakt, welcher die beiden tautomeren Fermentationsprodukte (−)-(S)-3,4-Dihydroxy-2,6,6-trimethyl-2-cyclohexen-1-on und (−)-(S)-3,6-Dihydroxy-2,4,4-trimethyl-2-cyclohexen-1-on enthält.

Durch Acetonisierung analog den entsprechenden Angaben in Beispiel 1 enthält man das (7aS)-7,7a-Dihydro-2,2,4,6,6-pentamethyl-1,3-benzodioxol-5(6H)-on mit einem Schmelzpunkt von 79−80° C; $[\alpha]_D = +105°$ (in Dioxan, c=0,102%), welches in Analogie zu den Angaben in Beispiel 1 in das (3S,3'S)-Astaxanthin übergeführt wird.

**Patentansprüche**

1. Verfahren zur Herstellung von racemischen oder optisch aktiven Polyenverbindungen, dadurch gekennzeichnet, daß man ein racemisches oder optisch aktives Diol der Formel

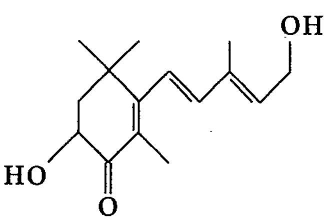

I

halogeniert und das erhaltene Halogenid mit einem Triarylphosphin umsetzt, und daß man erwünschtenfalls das erhaltene Phosphoniumsalz der allgemeinen Formel

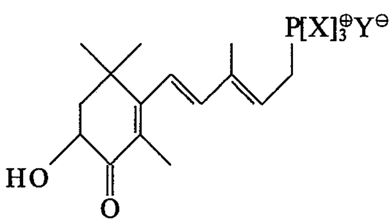

III

in der X einen Arylrest und Y Halogen bezeichnet, mit dem Dialdehyd der Formel

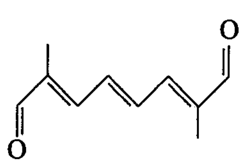

IV

zu racemischem oder optisch aktivem Astaxanthin kondensiert.

2. Verfahren nach Anspruch 1, dadurch gekennzeichnet, daß man das Diol der Formel I bromiert oder jodiert.

3. Eine Verbindung der Formel

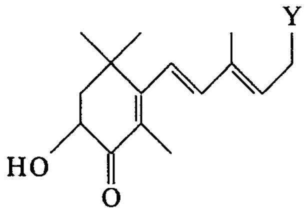

II

worin Y Halogen bedeutet, in racemischer Form und in Form der optischen Antipoden.

**Claims**

1. Process for the manufacture of racemic or optically active polyene compounds, characterized by halogenating a racemic or optically active diol of the formula

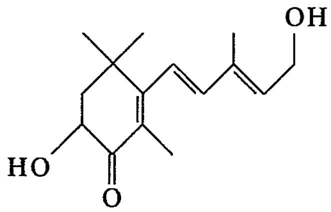

I

and reacting the halide obtained with a triarylphosphine, and, if desired, condensing the obtained phosphonium salt fo the general formula

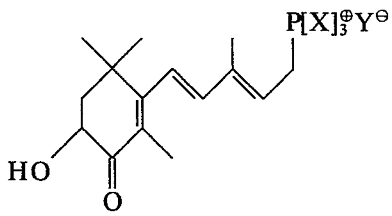

III

in which X denotes an aryl residue and Y denotes halogen, with the dialdehyde of the formula

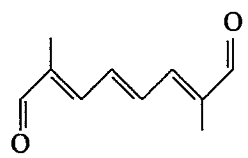

IV

to give racemic or optically active astaxanthin.

2. Process according to claim 1, characterized in that the diol of formula I is brominated or iodinated.

3. A compound of the formula

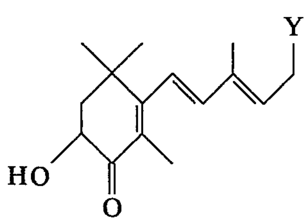

II

wherein Y signifies halogen, in racemic form and in the form of the optical antipodes.

**Revendications**

1. Procédé de préparation de composés polyéniques racémiques ou isomères optiques, caractérisé en ce que l'on halogène un diol racémique ou isomère optique de formule:

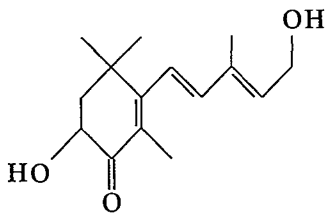

I

et on fait réagir l'halogénure obtenu avec une triarylphosphine, et si on le désire, on condense le sel de phosphonium obtenu de formule générale:

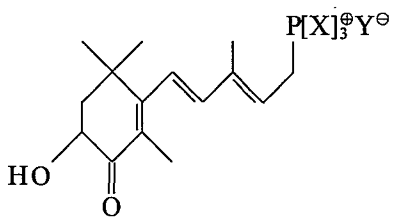

III

dans laquelle X représente un reste aryle et Y un halogène, avec le dialdéhyde de formule:

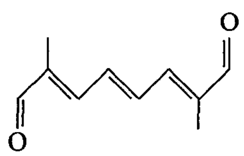

IV

formant ainsi une astaxanthine racémique ou isomère optique.

2. Procédé selon la revendication 1, caractérisé en ce que l'on brome ou l'on iode le diol de formule I.

3. Un composé de formule

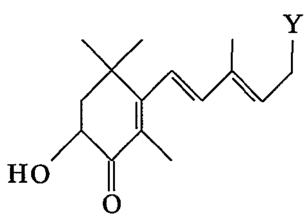

II

dans laquelle Y représente un halogène, sous forme racémique et sous forme des isomères optiques.